# EUROPEAN PATENT SPECIFICATION

(11) **EP 4 437 569 B1**
(45) Date of publication and mention of the grant of the patent: **01.10.2025**
(21) Application number: 22797887.1
(22) Date of filing: 24.10.2022
(51) Int. Cl.: H01B 1/24, A23L 29/00, A61K 8/92, A61K 8/31, A61Q 19/00

(54) **EDIBLE ELECTRICALLY CONDUCTIVE COMPOSITION**
ESSBARE ELEKTRISCH LEITFÄHIGE ZUSAMMENSETZUNG
COMPOSITION ÉLECTRIQUEMENT CONDUCTRICE COMESTIBLE

(30) Priority: 22.11.2021 IT 202100029498
(43) Date of publication of application: 02.10.2024
(73) Proprietor: Fondazione Istituto Italiano di Tecnologia, 16163 Genova (IT)
(72) Inventor: CATALDI, Pietro, 16123 Genova (IT); LAMANNA, Leonardo, 70044 Polignano a Mare (BA) (IT); CAIRONI, Mario, 20131 Milano (IT)
(74) Representative: Buzzi, Notaro & Antonielli d'Oulx S.p.A.
(86) International application number: PCT/IB2022/060201
(87) International publication number: WO 2023/089411

(56) References cited:
- WO-A1-2021/064453
- CA-A1- 3 106 795
- QURESHI DILSHAD ET AL: "Effect of carboxylated carbon nanotubes on physicochemical and drug release properties of oleogels", COLLOIDS AND SURFACES A: PHYSIOCHEMICAL AND ENGINEERING ASPECTS, ELSEVIER, AMSTERDAM, NL, vol. 610, 11 October 2020 (2020-10-11), XP086425446, ISSN: 0927-7757, [retrieved on 20201011], DOI: 10.1016/J.COLSURFA.2020.125695
- DHAL SOUMYASHREE ET AL: "Magnetic nanoparticle incorporated oleogel as iontophoretic drug delivery system", COLLOIDS AND SURFACES B: BIOINTERFACES, ELSEVIER AMSTERDAM, NL, vol. 157, 26 May 2017 (2017-05-26), pages 118 - 129, XP085152774, ISSN: 0927-7765, DOI: 10.1016/J.COLSURFB.2017.05.061
- FOWKES F M ET AL: "Oil-soluble surface-active copolymers of alkenes and polar monomers", JOURNAL OF COLLOID SCIENCE,, vol. 15, no. 6, 1 December 1960 (1960-12-01), pages 531 - 545, XP024209975, ISSN: 0095-8522, [retrieved on 19601201], DOI: 10.1016/0095-8522(60)90057-X

## Description

### Field of the invention

The description refers to edible electrically conductive compositions, particularly suitable, for example, for food quality monitoring and edible diagnostic applications.

### Background of the invention

"Edible electronics" is a new emerging field. The ultimate goal of edible electronics is to create devices that are safely edible without harmful impact on humans and can perform a specific task before or after ingestion. Edible electronics radically deviates from "ingestible electronics", which is instead made of conventional, silicon-based electronics, encapsulated in an inert, ingestible pill. Such pill has to be administered under strict supervision in a hospital because of risks of retention, and recollected after evacuation from the body. Edible electronics instead is fully made of edible materials, and is degraded (e.g., digested or metabolized) after performing its function. Applications of this electronic branch are envisioned in food quality monitoring (e.g., through food labels/tags) or smart electronic pills. To enable such disruptive technology, the first step is creating a library of materials (i.e., insulators, semiconductors, and conductors) that are the basic building block for eatable electronic technologies.

Electrical conductors specifically designed for being edible in large amounts can substitute the traditional electrodes used for standard and ingestible electronics, mainly built with inorganic materials, non-degradable, long-lasting, and sometimes poisoning humans and the environment imposing their recollection after expulsion.

To date, materials used as electrodes in electronics are metals such as magnesium, zinc, iron, tin, silver, gold. Unfortunately, these materials are pricey and not compatible with bulk and versatile production methods generally used, for example, in the polymer industry. According to the European Food Safety Agency, they can be safely ingested only in small amounts, in the range of µg-mg/kg body weight/day. They do not show any adhesive properties, and, in addition, some of them are not chemically inert, thus leading to the formation of undesired by-products when ingested.

Carbon-based conductive materials may offer solutions for ingestible conductors that can be safely ingested in hundreds of milligrams. Known carbon electronic materials, such as graphene, carbon nanotubes, carbon nanofibers, and organic conductors, however cannot be safely ingested.

D.. Qureshi et al. describe edible oleogels prepared from shea butter (SB) and wheat germ oil (WGO), mixed with carbon nanotubes:
D. Qureshi et al.: "Effect of carboxylated carbon nanotubes on physicochemical and drug release properties of oleogels", Colloids and Surfaces A: Physicochemical and Engineering Aspects, Elsevier, Amsterdam, NL, vol. 610, 11 October 2020 (2020-10-11), ISSN: 0927-7757, DOI: 10.1016/J.COLSURFA.2020.125695 .

Pure activated carbon (AC) instead is an electrically conductive material with a safe daily intake equal to or higher than 500 mg/kg body weight/day. Therefore, besides being low-cost, it is edible. However, AC is generally produced on a large scale in powder form and cannot be processed as-is for electrical application purposes. Moreover, AC powders do not satisfy the mechanical and adhesion properties required in many edible electronics applications such as monitoring of fruit ripening.

### Summary of the invention

The object of the present description is to provide edible electrically conductive compositions having a self-standing consistency, showing adhesion properties and, at the same time, stable electrical properties in air and in water-based liquids and solutions.

The object is achieved thanks to the subject matter recalled specifically in the ensuing claims, which are understood as forming an integral part of this disclosure.

One or more embodiments of the present description provide an edible electrically conductive composition, said composition comprising:
a) a matrix comprising at least one wax and at least one oil,
b) an edible electrically conductive material.

The composition may comprise at least one wax selected in the group consisting of beeswax, carnauba wax, candelilla wax, sugarcane wax, shellac, paraffin wax, crystalline wax, spermaceti wax, rice bran wax. Other known edible waxes may also be used.

The composition may comprise at least one oil selected in the group consisting of sunflower oil, corn oil, palm oil, margarine, butter, rapeseed oil, peanut oil, avocado oil, soybean oil, olive oil. Other known edible oils may also be used.

In one or more embodiments, the edible electrically conductive material is activated carbon (AC).

The electrically conductive material may comprise particles in the range of 100 nanometer (nm) and 100 micrometer (µm).

In one or more embodiments, the wax and the oil may be present in the composition in a weight ratio comprised between 0.05:1 and 1:0.05.

The weight ratio between the edible electrically conductive material and the matrix (comprising at least one wax and at least one oil) may be comprised between 0.1:1 and 1:1, preferably between 0.2:1 and 0.6:1

The description also refers to a method for providing edible electrically conductive formations on a substrate, the method comprising applying the edible electrically conductive composition on the substrate. The substrate may vary according to the intended application and may be an edible substrate (e.g., fruit) or a not edible substrate (e.g., polymers, glasses, metals).

### Brief description of the figures

One or more embodiments will be described, purely by way of non-limiting example with reference to the annexed figures, listed below.

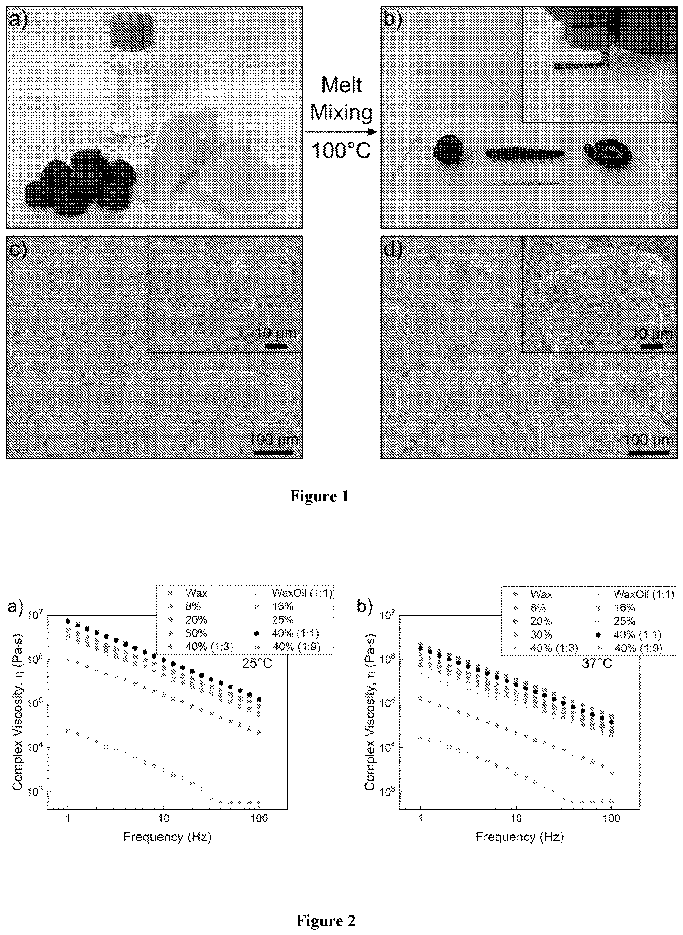

- Figure 1 shows, in panel a), the raw materials comprised in the composition herein disclosed: oil, wax, and activated carbon (AC). Panel b) shows the pliability of the edible composition and provides evidence of its potential application in direct ink writing techniques (inset). Panels c) and d) show scanning electron microscopy (SEM) images of a sample composition comprising a matrix of pure wax and oil in a weight ratio 1:1 and a sample composition comprising a matrix of wax and oil in a weight ratio 1:1 and adding AC in a weight amount of 30% relative to the weight of the matrix, respectively;
- Figure 2 shows the rheological feature (viscosity, η) of the compositions herein disclosed. Panels a) and b) show the viscosity of i) pure wax (Wax), ii) wax-oil oleogel at a 1:1 ratio (WaxOil (1:1)), iii) samples realized adding different amounts of AC (from 0 to 40 wt.%) at 25°C and 37°C, respectively. Tunability of the viscosity may be achieved by varying the amount of AC included in the sample and/or changing the wax:oil weight ratios. This last approach was demonstrated for the sample with 40% AC and with a wax:oil ratio of 1:1, 1:3, and 1:9 (labelled 40% (1:1), 40% (1:3), and 40% (1:9), respectively);
- Figure 3 shows SEM images of sample compositions comprising 40% AC added in a matrix comprising wax and oil in a weight ratio of 1:1 (40%(1:1)) and 1:3 (40%(1:3)).
- Figure 4 shows the adhesive shear strength of comparative sample compositions on polymethylmethacrylate (PMMA) and glass substrates. For the nomenclature see Figure 2;
- Figure 5 shows the electrical resistivity as a function of the AC added to the oil:wax matrix (ratio 1:1) (left panel); the electrical resistivity values versus time of immersion normalized to the initial value R₀ are shown in the right panel ;
- Figure 6 refers to a proof of concept label of an apple surface for fruits electrical impedance spectroscopy (EIS). Panels a)-c) are photos showing of the steps of assembling the composition as a label on an apple surface; panel d) shows its cross-section. Panel e) provides the evidence of the adhesion property of the composition (the label supporting the weight of an apple);
- Figure 7 shows an histogram of the time dependent impedance of the apple at 100 kHz recorded with the edible label/tag composition (gray column) and by using silver paint (black column).

### Detailed description

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. The headings provided herein are for convenience only and do not interpret the scope or meaning of the embodiments.

The edible electrically conductive composition herein disclosed only comprises food-grade components, safely ingestible without exerting adverse effects.

One or more embodiments of the present description provide an edible electrically conductive composition, said composition comprising:
a) a matrix comprising at least one wax and at least one oil,
b) an edible electrically conductive material.

The composition may comprise at least one wax selected in the group consisting of beeswax, carnauba wax, candelilla wax, sugarcane wax, shellac, paraffin wax, crystalline wax, spermaceti wax, rice bran wax. Other known edible waxes may also be used.

The composition may comprise at least one oil selected in the group consisting of sunflower oil, corn oil, palm oil, margarine, butter, rapeseed oil, peanut oil, avocado oil, soybean oil, olive oil. Other known edible oils may also be used.

In one or more embodiments, the matrix may consist of at least one wax and at least one oil. The matrix may be in a form of an oleogel matrix.

The edible electrically conductive material may preferably be activated carbon (AC).

The electrically conductive material may comprise particles having a size comprised between 100 nm and 100 µm.

The edible electrically conductive material may be dispersed in the matrix.

In one or more embodiments, the wax and the oil may be present in the composition in a weight ratio comprised between 0.05:1 and 1:0.05.

The weight ratio between the edible electrically conductive material and the matrix (comprising at least one wax and at least one oil) may be comprised between 0.1:1 and 1:1, preferably between 0.2:1 and 0.6:1.

The composition may be in a form of a paste.

The description also refers to a method for providing edible electrically conductive formations on a substrate, the method comprising applying the edible electrically conductive composition on the substrate. The description also provides a substrate at least partially coated with the edible electrically conductive composition. The substrate may vary according to the intended application and may be an edible substrate (e.g., fruit) or a not edible substrate (e.g., polymers, glasses, metals). Applied on a substrate, the composition may be used to electrically contact different electronic components.

In the following sections, the Inventors provide evidence that the composition may be shaped on demand and be direct ink writable (as shown in Figure 1, panel b)).

The composition may be also compatible with other techniques, such as extrusion and injection molding and may be 3D printed through fused deposition modeling.

The edible electrically conductive composition herein disclosed may be produced by a method comprising the following steps:
i) heating at least one wax,
ii) adding at least one oil to heated wax and mixing to create a matrix,
iii) adding the edible electrically conductive material to the matrix and mixing the components obtaining the edible electrically conductive composition.

In one or more embodiments, the method may comprise the following steps:
i) providing at least one oil,
ii) heating at least one wax and adding at least one oil to the heated wax to create a matrix,
iii) adding the edible electrically conductive material to the matrix and mixing the components obtaining the edible electrically conductive composition.

The heating step may be carried out at a temperature comprised between 50°C and 250°C. The heating step allows achieving the melting of the wax. The other two steps may also be carried out at the same temperature (comprised between 50°C and 250°C).

Advantageously, the edible electrically conductive composition is free of solvents.

Moreover, the temperature range used for the production method leaves intact the chemical signature of wax and oil once the composition is achieved and, at the same time, ensures a complete melting of the wax.

Figure 1 shows, in panel c), the micromorphology of a composition comprising wax and oil in a weight ratio 1:1 ("WaxOil (1:1))". This composition is an oleogel and the gelation mechanism creates micrometric plate-like wax assemblies, which aggregate to form 3D networks of wax crystals capable of entrapping liquid oils.

The oleogel nature of the matrix guarantees a self-standing property of the composition and, at the same time, the pliability and conformability proper of a paste.

Adding the edible electrically conductive material, preferably AC, as shown in Figure 1d), on the one hand, leads to a more uneven surface; on the other hand, due to the AC intrinsic porosity that "dries and sucks" the oil, the wax microcrystals adhere to the AC particles. Thus, by including AC as electrically conductive material to the matrix comprising wax and oil the tuning of the mechanical and adhesion properties of the composition may be achieved, imparting at the same time electrical conductivity (see next sections).

The description also refers to a method for providing electrically conductive formations on a substrate, the method comprising applying the edible electrically conductive composition on the substrate.

The description also provides a substrate at least partially coated with the edible electrically conductive composition. The substrate may vary according to the intended application and may be an edible substrate (e.g., fruit) or a not edible substrate (e.g., polymers, glasses, metals).

As shown in the following section, the electrically conductive composition herein disclosed comprises food-grade materials ingestible in large amounts (i.e., ≥ mg/kg body weight/day) without any potential adverse impact on human health. Moreover, the Inventors have shown that: 1) keeping the oil:wax ratio constant and varying the content of the electrically conductive material leads to a tunable electrical conductivity, rheology, and adhesion; 2) keeping the electrically conductive materials content constant and varying the oil:wax ratio leads to a tuneable rheology and adhesion properties with constant electrical conductivity; 3) oil:wax:electrically conductive materials ratios change enable a fine-tuning of the electrical conductivity, rheology, and adhesion.

The edible conductive compositions maintain the electrical conductivity unchanged in contact with water-based liquids and solutions.

In the following some non limiting examples of different embodiments of the composition object of the instant description will be provided.

### Examples

### Method for producing the edible electrically conductive composition

The composition is produced by first heating and melting the waxes (at least one of, for example, beeswax, carnauba wax, candelilla wax, sugarcane wax, shellac, paraffin wax, crystalline wax, spermaceti wax, rice bran wax, and other well-known edible wax) and mixing them with oils (at least one of, for example, sunflower oil, corn oil, palm oil, margarine, butter, rapeseed oil, peanut oil, avocado oil, soybean oil, olive oil, and other well-known edible oil) to form the oleogel matrix. Secondly, activated carbon (AC, a.k.a. activated charcoal and vegetable carbon, E 153) is added and mixed to the oleogel matrix to achieve the conductive composition. The heating step may be carried out at a temperature comprised between 50°C and 250°C. The other steps may also be carried out at the same temperature (comprised between 50°C and 250°C).

For different experimental analysis, compositions have been produced as disclosed above, using different wax:oil weight ratios, ranging from 0.05:1 to 1:0.05. Afterward, the required amount of AC as electrically conductive material was added and mixed at a temperature range of 50°C-250°C. The weight ratio between the edible electrically conductive material and the matrix (comprising at least one wax and at least one oil) may be comprised between 0.1:1 and 1:1, preferably between 0.2:1 and 0.6:1.

### Tunable rheology and self-standing consistency

The ability to adapt and tune the rheology of the edible conductive composition herein disclosed through precise variations of the matrix and of the electrically conductive material can provide materials producible with many manufacturing techniques, with multiple functionalities, and easily adaptable to a specific application. The rheological properties of the pure wax-oil matrix (in a weight ratio 1:1) and the composition obtained by adding to this matrix different amounts of AC (from 0 to 30 wt.%, labelled simply referring to the wt.% of the AC added to the wax and oil matrix, were tested as an example. The sample with 40% AC were tested varying also the wax:oil ratio (in particular 1:1, 1:3, and 1:9 wax:oil ratio, labelled 40% (1:1), 40% (1:3), and 40% (1:9), respectively).

For example, if the weight of the wax and oil matrix was 100 g, the sample labelled 30% AC contained 30g of AC, and the matrix was composed of 50g of wax and 50 g of oil. As another example, if the weight of the wax and oil matrix was 100 g, the sample labeled 40% (1:9) contained 40 g of AC, and the weight of the matrix was composed of 10 g of wax and 90 g of oil.

Frequency sweep analysis was performed between 1 Hz and 100 Hz at ambient temperature (25°C) and at 37°C to simulate a temperature similar to that of the human body. All the tested compositions comprising AC showed a solid gel-like viscoelastic behavior. Interestingly, the η was fine-tuned by varying the amount of AC comprised in the composition and decreased with increasing frequency, as shown in Figure 2a and Figure 2b.

At 1 Hz, the η ranged (at 25°C and 37°C) from ~ 3.0•10⁶ [Pa s] to 7.3•10⁶ [Pa s] and from 4.9•10⁵ [Pa s] to 1.8•10⁶ [Pa s] for the composition only comprising the wax-oil matrix (weight ratio 1:1) and for the 40% (1:1) sample, respectively.

Compositions comprising a higher amount of electrically conductive material (filler, AC for example) may appear with high viscosity and not meltable; this may favor the production of a brittle and powdery composition.

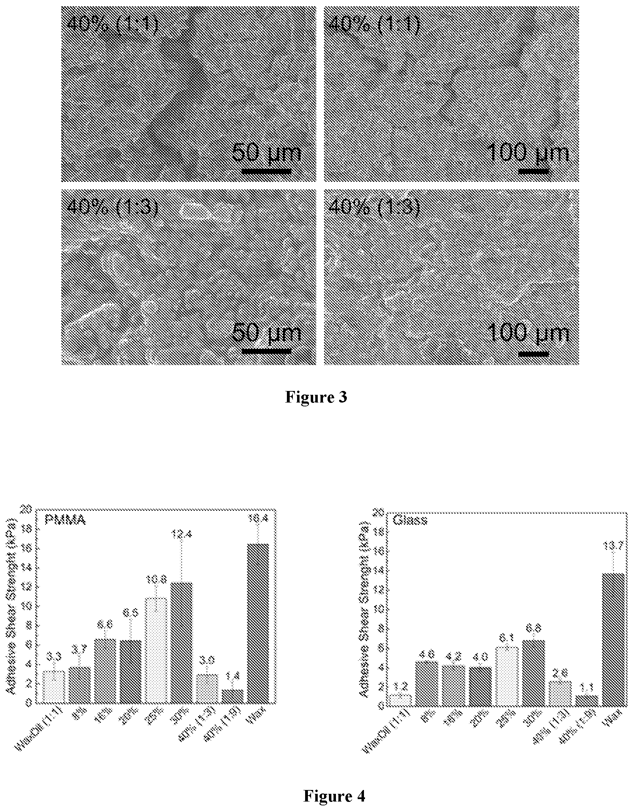

The compositions 40% (1:1) showed a "paste-powder" form macroscopically and the abovementioned aspects. Microscopically, the brittleness of the composition was confirmed by SEM images, which showed a texture with multiple micrometric cracks, as shown in Figure 3. This can be tuned/overcome by changing the formulation of the edible composition. Indeed, the tuning of the rheological properties for this sample composition containing 40% AC was achieved by changing the wax and oil weight ratio, which improved the composition's melt-miscibility, softness, and conformability. For example, at 25°C and 37°C, as shown in Figure 2, changing the oil:wax ratio from 1:1 to 1:3 and 1:9 (sample 40% (1:3) and 40% (1:9), respectively) was effective in decreasing the complex viscosity of one and two orders of magnitude, respectively. Microscopically, as shown in Figure 3, the cracks were absent in the composition comprising AC in an amount of 40% by weight and oil:wax in a 1:3 weight ratio (labelled 40% (1:3)). This composition recovered the self-standing nature and adhesion, as shown in Figure 4.

### Tunable Adhesion

An important feature of the composition herein disclosed is the capability of adhesion to diverse surfaces. Adhesion on a standard plastic substrate (polymethyl methacrylate, PMMA) and glass was tested as an example. These tests were performed on the the sample described and labelled in the previous section, as shown in Figure 4.

In particular, shear adhesive strength tests demonstrated that the adhesion strength of the wax-oil matrix could be tuned by the inclusion of different amounts of AC inside the composite formulation. Indeed, the pure oleogel "WaxOil (1:1)" displayed an adhesive strength on PMMA of ~3.3 kPa; the compositions comprising the wax and oil in a weight ratio 1:1 in combination with AC in different amount (from 8% to 30 wt.%) showed an enhanced adhesion from ~3.7 to ~12.4 kPa, a value close to pure wax that reached a value of ~16.4 kPa.

40% (1:1) sample showed too low adhesive performances to be evaluated; interestingly, by tuning the wax to oil weight ratio to 1:3 and 1:9 (sample 40% (1:3) and 40% (1:9), respectively), an adhesion strength of ~3.0 and ~1.4 kPa, respectively, has been restored, as shown in Figure 4.

The same behavior was measured on glass substrates, although with slightly lower adhesive strength. The tunable adhesive strength of the composition has been achieved thanks to the porosity of the AC filler that "dries" the oil component, enhancing its adherence and maintaining a self-standing structure. In the 40% (1:1) composition, the dry phase becomes predominant and the sample's powdery nature negatively influences its adhesive properties. Changing the weight ratio between wax and oil to 1:3 and 1:9 (sample 40% (1:3) and 40% (1:9), respectively) allows the recovery of adhesive properties. The measured adhesive strength values are comparable, for example, with polymer-based degradable adhesives used for surgery and tissue engineering and polyurethane-based adhesive for wood.

### Electrical characterization in air and underwater

Including electrically conductive fillers inside insulating matrixes can induce electrical conductivity on the resulting composition. The minimum filler loading required to switch the composition from an insulator to a conductor is known as the electrical percolation threshold.

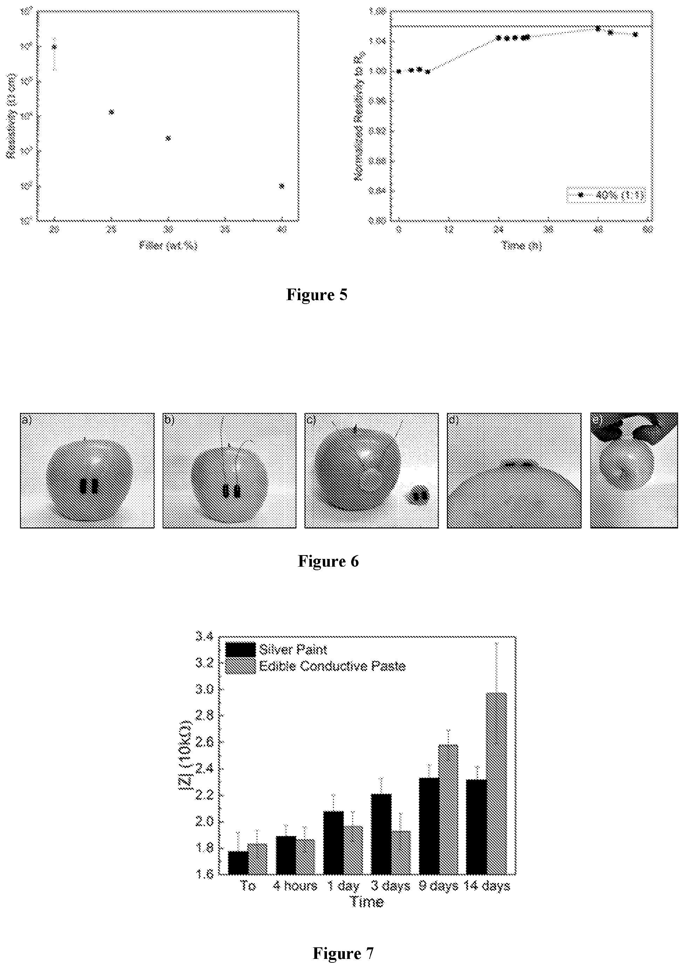

The resistivity of the edible paste was measured for different amounts of AC filler incorporated inside the wax and oil (oleogel) matrix. At a filler loading until 16% AC the composition was an insulator. At 20 wt.% AC, the resistivity of the composition was ~ 1 MΩ·cm, reaching electrical percolation. Increasing the AC content to 40% by weight, the composition resistivity reached values of 101±2 Ω·cm, as shown in Figure 5 (left panel). The resistivity values are comparable with other reports that employ solely AC (range of resistivity 0.5-1000 Ω·cm) but, at the same time, the composition ensures pliability, conformability, and compatibility with large-scale production methods proper of the composite industry.

For practical application, a desirable property for an ingestible conductor is the stability of the electrical conduction properties even in contact with water-based liquids. Thus, a composition comprising AC in an amount of 40% by weight relative to the oleogel was immersed in water for more than 50 hours and their electrical resistance variation was monitored. Throughout this period, the resistivity changed less than 6% of the initial value, as shown in Figure 5 (right panel). This factor revealed a shallow interaction of the samples with water. Thus, the stability of the electrical feature of the presented invention in contact with water-based liquid or solutions is improving the state-of-the-art, which is based on transient components that dissolve in water or ionic conductors that change their conductivity depending on hydration.

### Electrical impedance measurement (EIS)

Monitoring the maturation of fruits through electrical impedance spectroscopy (EIS) is a disruptive technology to improve the agricultural sector's efficiency and reduce food waste. EIS on fruit can potentially enable quick, on the ground, quantitative, and portable analysis of fruit degree of maturation, substantially improving commercially available devices, which mainly monitor their ripening through smart labels on food packaging.

Building an edible label directly attachable to the fruits would enable monitoring the fruits production chains, from the tree to the table. Crucial aspects to realize such technology involve the need to achieve edible, adhesive, low-cost electrodes.

State of the art electrodes exploit Ag/AgCl or other metallic ones that need electrolyte gels applied between them and fruits to achieve a satisfying adhesion. These devices show drawbacks if applied to food chain supply monitoring. Indeed, the Ag/AgCl electrodes are pricey, and their acceptable daily intake is low (µg/Kg body weight/day), making Ag/AgCl electrodes unsuitable for this technology.

A proof of concept eatable electrical impedance measurement (EIS) label has been realized, as shown in Figure 6, panels a) to c). Two conductive electrodes were stuck on the apple, they were wired and then encapsulated with pure beeswax. For this purpose, the composition selected was 40% (1:3) as defined in the previous sections. The encapsulating wax layer protects and "freezes" the system, enhancing its adhesion to the fruit. The electrodes were fully compliant on the apple surface, as shown in Figure 6, panel d). The obtained tag thoroughly adhered to the fruit to support the weight of the apple itself without detaching (see Figure 6, panel e)).

The EIS recorded through the edible conductive composition has been compared with silver compositions since metallic electrodes are commonly used for fruit EIS. The impedance (|Z|) of the apple measured at 100 kHz through the edible device and the reference silver paint, respectively, are presented in Figure 7. In 14 days, the impedance increased similarly for the composition herein disclosed and the standard silver electrodes, suggesting that the developed material can be as efficient as state-of-the-art electrodes for EIS on food. The measurement shows that the label made with the edible composition herein disclosed gives a value of |Z| of the apple of ~ 18 kQ at the starting time and reaches a value of ~ 30 kΩ after 14 days. A similar starting value is recorded with the silver paint and reaches a value of ~ 23 kΩ after 14 days.

The edible electrically conductive composition is low-cost, can be shaped on request, and is compatible with many thermoplastic polymer-manufacturing techniques (e.g., 3D printing, extrusion, and injection molding) since it is endowed with on-demand rheological features. It is also ideal for an industrial scale-up and shows tunable adhesion properties on many surfaces by varying the weight ratios of its components. The composition also shows constant electrical properties even underwater, a salient characteristic for electrodes that are designed for ingestion and contact with gastric fluids. The composition may find application in food monitoring, and pharmaceutics such as smart pills (for example as a conductive (edible) component of the pill) and drugs delivery systems. The project leading to this application has received funding from the European Union's Horizon 2020 research and innovation programme under grant agreement No 864299.

## Claims

1. Edible electrically conductive composition comprising:
a) a matrix comprising at least one wax and at least one oil,
b) an edible electrically conductive material.

2. Edible electrically conductive composition according to claim 1, wherein said at least one wax is selected in the group consisting of beeswax, carnauba wax, candelilla wax, sugarcane wax, shellac, paraffin wax, crystalline wax, spermaceti wax, rice bran wax.

3. Edible electrically conductive composition according to claim 1 or claim 2, wherein said at least one oil is selected in the group consisting of sunflower oil, corn oil, palm oil, margarine, butter, rapeseed oil, peanut oil, avocado oil, soybean oil, olive oil.

4. Edible electrically conductive composition according to any preceding claims, wherein said edible electrically conductive material is activated carbon.

5. Edible electrically conductive composition according to any preceding claims, wherein said electrically conductive material comprises particles having a size comprised between 100 nm and 100 µm.

6. Edible electrically conductive composition according to any preceding claims, wherein said at least one wax and at least one oil are present in the composition in a weight ratio comprised between 0.05:1 and 1:0.05.

7. Edible electrically conductive composition according to any preceding claims, wherein the weight ratio between the edible electrically conductive material and the matrix comprising at least one wax and at least one oil may be comprised between 0.1:1 and 1:1, preferably between 0.2:1 and 0.6:1.

8. Method for providing electrically conductive formations on a substrate, the method comprising applying an edible electrically conductive composition according to any of the preceding claims 1 to 7 on said substrate.

9. A substrate at least partially coated with an edible electrically conductive composition according to any one of claims 1 to 7.

## Patentansprüche

1. Essbare elektrisch leitfähige Zusammensetzung, umfassend:
a) eine Matrix, die mindestens ein Wachs und mindestens ein Öl umfasst,
b) ein essbares elektrisch leitfähiges Material.

2. Essbare elektrisch leitfähige Zusammensetzung nach Anspruch 1, wobei das mindestens eine Wachs aus der Gruppe ausgewählt ist, die aus Bienenwachs, Carnaubawachs, Candelillawachs, Zuckerrohrwachs, Schellack, Paraffinwachs, kristallinem Wachs, Spermacetiwachs und Reiskleiewachs besteht.

3. Essbare elektrisch leitfähige Zusammensetzung nach Anspruch 1 oder Anspruch 2, wobei das mindestens eine Öl aus der Gruppe ausgewählt ist, die aus Sonnenblumenöl, Maisöl, Palmöl, Margarine, Butter, Rapsöl, Erdnussöl, Avocadoöl, Sojaöl und Olivenöl besteht.

4. Essbare elektrisch leitfähige Zusammensetzung nach einem der vorstehenden Ansprüche, wobei das essbare elektrisch leitfähige Material Aktivkohle ist.

5. Essbare elektrisch leitfähige Zusammensetzung nach einem der vorstehenden Ansprüche, wobei das elektrisch leitfähige Material Partikel mit einer Größe zwischen 100 nm und 100 µm umfasst.

6. Essbare elektrisch leitfähige Zusammensetzung nach einem der vorstehenden Ansprüche, wobei das mindestens eine Wachs und das mindestens eine Öl in der Zusammensetzung in einem Gewichtsverhältnis zwischen 0,05:1 und 1:0,05 vorliegen.

7. Essbare elektrisch leitfähige Zusammensetzung nach einem der vorstehenden Ansprüche, wobei das Gewichtsverhältnis zwischen dem essbaren elektrisch leitfähigen Material und der Matrix, die mindestens ein Wachs und mindestens ein Öl umfasst, zwischen 0,1:1 und 1:1, vorzugsweise zwischen 0,2:1 und 0,6:1 liegen kann.

8. Verfahren zum Bereitstellen elektrisch leitfähiger Formationen auf einem Substrat, wobei das Verfahren das Aufbringen einer essbaren elektrisch leitfähigen Zusammensetzung gemäß einem der vorstehenden Ansprüche 1 bis 7 auf das Substrat umfasst.

9. Substrat, das zumindest teilweise mit einer essbaren elektrisch leitfähigen Zusammensetzung gemäß einem der Ansprüche 1 bis 7 beschichtet ist.

## Revendications

1. Composition comestible électriquement conductrice comprenant :
a) une matrice comprenant au moins une cire et au moins une huile,
b) un matériau comestible électriquement conducteur.

2. Composition comestible électriquement conductrice selon la revendication 1, dans laquelle ladite au moins une cire est choisie dans le groupe constitué par la cire d'abeilles, la cire de carnauba, la cire de candélilla, la cire de canne à sucre, la gomme laque, la cire de paraffine, la cire cristalline, la cire de spermacéti, la cire de son de riz.

3. Composition comestible électriquement conductrice selon la revendication 1 ou la revendication 2, dans laquelle ladite au moins une huile est choisie dans le groupe constitué par l'huile de tournesol, l'huile de maïs, l'huile de palme, la margarine, le beurre, l'huile de colza, l'huile d'arachide, l'huile d'avocat, l'huile de soja, l'huile d'olive.

4. Composition comestible électriquement conductrice selon l'une quelconque des revendications précédentes, dans laquelle ledit matériau comestible électriquement conducteur est le charbon activé.

5. Composition comestible électriquement conductrice selon l'une quelconque des revendications précédentes, dans laquelle ledit matériau électriquement conducteur comprend des particules ayant une taille comprise entre 100 nm et 100 µm.

6. Composition comestible électriquement conductrice selon l'une quelconque des revendications précédentes, dans laquelle lesdites au moins une cire et au moins une huile sont présentes dans la composition en un rapport en poids compris entre 0,05/1 et 1/0,05.

7. Composition comestible électriquement conductrice selon l'une quelconque des revendications précédentes, dans laquelle le rapport en poids entre le matériau comestible électriquement conducteur et la matrice comprenant au moins une cire et au moins une huile peut être compris entre 0,1/1 et 1/1, de préférence entre 0,2/1 et 0,6/1.

8. Procédé pour disposer des formations électriquement conductrices sur un substrat, le procédé comprenant l'application d'une composition comestible électriquement conductrice selon l'une quelconque des revendications 1 à 7 sur ledit substrat.

9. Substrat au moins partiellement revêtu d'une composition conductrice électriquement comestible selon l'une quelconque des revendications 1 à 7.
